# EUROPÄISCHE PATENTANMELDUNG

(11) **EP 0 853 946 A1**
(43) Veröffentlichungstag der Anmeldung: **22.07.1998**
(21) Anmeldenummer: 96120822.0
(22) Anmeldetag: 23.12.1996
(51) Int. Cl.: A61K 39/35, A61K 39/385, A61K 39/395

(54) **Anti-Allergene Zusammensetzung**

(71) Anmelder: BOEHRINGER MANNHEIM GMBH, 68298 Mannheim (DE)
(72) Erfinder: Lemke, Hilmar, Prof. Dr., D-24239 Achterwehr (DE); Opitz, Uta, Dr., D-69469 Weinheim (DE)

(57) **Zusammenfassung**

Die Erfindung betrifft Antigen-Antikörper-Komplexe, die bei der Prophylaxe und Therapie von allergenen Erkrankungen eingesetzt werden.

## Beschreibung

Die Erfindung betrifft Antigen-Antikörper-Komplexe, die bei der Prophylaxe und Therapie von allergenen Erkrankungen eingesetzt werden.

Unter allergenen Erkrankungen werden Andersempfindlichkeiten mit veränderter immunologischer Reaktionslage verstanden. Allergene lösen spezifisch Zustandsänderungen der Organismus aus, die eine Voraussetzung für die Entstehung pathogener Immunphänomene ist. Solche Zustandsänderungen können auf dem Vorhandensein von Antikörpern (Ak) oder von aktivierten T-Zellen beruhen.

Allergene sind allergene Erkrankungen auslösende Antigene (z.B. Proteine, Haptene, Haptide). Sie können nach der Art des Eindringens und Wirksamwerdens in verschiedenen Klassen eingeteilt werden (Inhalationsallergene, Ingestionsallergene, Kontaktallergene, Injektionsallergene, Infektionsallergene). Allergien werden in solche vom Soforttyp (Typ I bis III) und vom verzögerten Typ (Typ IV) eingeteilt.

Bei Typ I-Allergien kommt es zu Reaktionen zwischen zellständigem Antikörper (IgE) und Allergen mit Freisetzung von Mediatoren aus basophilen Granulozyten bzw. Mastzellen. Sekundär werden auch andere Zellen einbezogen. Resultierende Krankheitsbilder sind u.a. Asthma bronchiale, Rhinitis pollinosa, Konjunctivitis allergica, anaphylaktischer Schock, Urticaria, allergische Reaktionen (z.B. auf Insektenstich, Arzneimittel wie Progesteron, Östrogen, Nahrungsmittel), endogenes Ekzem.

Neben der klassischen Einteilung in Typ I- bis Typ IV-Allergien wird in der Literatur noch ein Typ V beschrieben, bei dem es sich um eine Immunreaktion gegen biologisch aktive Determinanten handelt. Diese Reaktionen sind von erheblicher klinischer Relevanz, die von physiologischer Regulation (Antiidiotyp Ak) bis zu pathogenen Reaktionen reichen. Als Antigen können Zellrezeptoren oder azelluläre biologisch aktive Strukturen (Hormone, Enzyme, intrinsic-Faktor, etc.) wirken. Diese Ak können stimmulierend oder inhibierend wirken.

Die Desensibilisierung oder Hyposensibilisierung gilt momentan neben der Verabreichung von immunsuppressiven Substanzen als Therapiekonzept gegen allergische Erkrankungen. Bei der Immunsuppressionen treten neben unerwünschten Nebenwirkungen der immunosuppressiven Substanzen auch z.T. eine Erhöhung der Toleranz des Patienten gegen diese Substanzen auf was zu stetig steigenden Wirkstoffapplikationen führt.

Bei der Desensibilisierung wird das Allergen in allmählich steigender Dosis verabreicht, mit dem Ziel eine bestehende Sensibilisierung zu beseitigen oder zu verhindern. Praktisch bedeutsam ist die Desensibilisierung bei der Behandlung atopischer Erkrankungen. Bei Typ IV-Allergien wird ihr Wert diskutiert. Bei Allergien vom Soforttyp wird sie breit eingesetzt. Die Erfolgschancen schwanken zwischen 50% und 90%. Die Patienten werden häufig behandelt und mögliche Nebenwirkungen gehen von Lokalreaktionen über Provokation von Schüben der Grundkrankheit bis zu anaphylaktischen Allgemeinreaktionen.

Durch die hier vorliegende Erfindung können die Nachteile von bisherigen Behandlungen von Allergien beseitigt werden. Es wurde überraschend gefunden, daß haptenisiertes Allergen (hAlleg) zusammen mit Hapten-spezifischen Antikörpern (hAk) zu einer Desensibilisierung gegen das Allergen führen. Die Antikörper können polyklonal oder monoklonal sein. Die hAk können aus einer nicht humanen Quelle stammen (z.B. Maus) sind dann aber bevorzugt humanisierte Ak. Alternativ können humane Antikörper (z.B. von menschlichen Zellinien oder transgenen Quellen) verwendet werden. Die hAks sind bevorzugt Antikörper der Klasse IgG, besonders bevorzugt der Subtypen IgG1, IgG2a und IgG2b der Maus. In Analogie gilt dasselbe auch bei den kotrespondierenden humanen IgG-Klassen. Es kann die Desensibilisierung sowohl mit dem hAk-hAllerg-Komplex durchgeführt werden oder durch getrennte Gabe von hAk und hAllerg. Die Desensibilisierung spricht sowohl bei der Gabe von mehreren verschiedenen hAk's gegen das gleiche Hapten zusammen mit einem haptenisierten Allergen an, als auch durch die Gabe eines einzigen hAk's mit hAllerg.

Diese neue Art der Desensibilisierung ist auch kommerziell interessant, da für eine Vielzahl von Allergenen nur ein hAk nötig ist, der im technischen Maßstab herstellbar ist. Die Kopplung der verschiedenen Allergene mit einen Hapten kann in bekannter Weise durchgeführt werden.

Eine weitere Ausführungsform der Erfindung betrifft die Gabe von haptenisiertem Carrier, der in vivo die Produktion von hAks bedingt. Bei anschließender Gabe von haptenisierten Allergenen findet die Bildung des hAk hAllerg-Komplexes in vivo statt. Eine anschließende Desensibilisierung wird dann bedingt. Der Carrier kann dabei selbst ein Allergen, ein anderes Antigen oder ein neutraler Carrier sein.

Antigen-Antikörper-Komplexe (Immunkomplexe) werden in vivo als Teil der Immunantwort gebildet. Immunkomplexe werden auch verwendet, um die Immunregulation zu untersuchen.

So induziert eine Immunisierung mit Immunkomplexen nur eine stark verminderte Primärantwort, die Sekundärantwort ist aber trotzdem entweder normal (Saffold J. W. et al. J. Immunol. 107: 1213-1225 (1971); Tite J.P. et al. Immunology 34: 1097-1107 (1978) oder sogar verstärkt (Taylor et al. Nature 281: 488-490 (1979)).

In der vorliegenden Erfindung wird dagegen eine neue Art der Desensibilisierung mit Allergen-Antikörper-Komplexen nachgewiesen. Dabei können auch anstelle von Allergen-Antikörper-Komplexen Allergen und spezifischer Antikörper getrennt und sogar allergen-spezifischer Antikörper allein für die Behandlung allergener Erkrankungen eingesetzt werden.

Dies bedeutet, daß bei weit verbreiteten Allergenen auf die Haptenisierung der Allergene auch verzichtet werden kann und direkt Allergen/Antiallergen-Antikörper-Komplexe zur Desensibilisierung eingesetzt werden. Ebenso können Antiallergen-Antikörper allein zur Desensibilisierung eingesetzt werden.

Um die erfindungsgemäßen hAk und hAllerg herzustellen, kann nach folgenden beispielhaft skizzierten Schema vorgegangen werden: Das spezifische induzierende Allergen wird durch entsprechende, bekannte Teste ermittelt. Das Allergen wird mit einem Hapten gekoppelt. Antihapten Antikörper werden nach bekannten Techniken produziert oder falls schon bekannt identifiziert. hAllerg und hAk werden in der Behandlung als Komplex oder getrennt eingesetzt. Bei der Verwendung von Komplexen zwischen Allergen und spezifischen Antikörper wird das Verfahren entsprechend modifiziert.

Beispiele für Haptene sind:
Fluoreszein-Isothiocyanat
Phenyloxazolon
Dinitrophenol
Digoxigenin
Biotin
Fluoreszein

### Experimenteller Teil

Als Modellsystem für Beispiele 1 bis 5 werden CBA-Mäuse verwendet, als Modellantigen PLA₂.

### Beispiel 1:

### Suppression der IgE-anti-PLA₂-Immunantwort nach Immunisierung mit PLA₂-IgG-anti-PLA₂-Komplexen.

CBA/J-Mäusen wurden allein 0,1 µg PLA₂ verabreicht [adsorbiert an 0,2 mg Al(OH)₃ - wie auch bei allen folgenden Gruppen] (Gruppe 1), oder dieselbe Dosis PLA₂ mit einem monoklonalen IgG2b-anti-PLA₂ Antikörper (molares Verhältnis 1:10; Gruppe 2), oder mit PLA₂ mit einem monoklonalen IgG1-anti-PLA₂ (molares Verhältnis 1:10; Gruppe 3), oder mit PLA₂ komplexiert mit einem 1:1 Gemisch des IgG1 - und IgG2b-Antikörpers (molares Ag-Ak-Verhältnis 1:10; Gruppe 4), oder den CBA/J-Mäusen wurde zuerst eine Mischung beider Antikörper injiziert, bevor sie 2 h später mit derselben Dosis von 0,1 mg PLA₂ i.p. immunisiert wurden. Nach 6 Wochen wurde im Serum der Tiere die Menge der Antikörper gegen PLA₂ vom IgE-Isotyp mittels ELISA bestimmt.

Es zeigte sich, daß die Immunisierungen mit allen gewählten Formen von Antigen-Antikörper-Komplexen eine Suppression der IgE-anti-PLA₂ Immunantwort bewirkten. Die stärkste Suppression wurde mit IgG2b-anti-PLA₂ Antikörpern erzielt (Gruppe 2 und Gruppe 5).

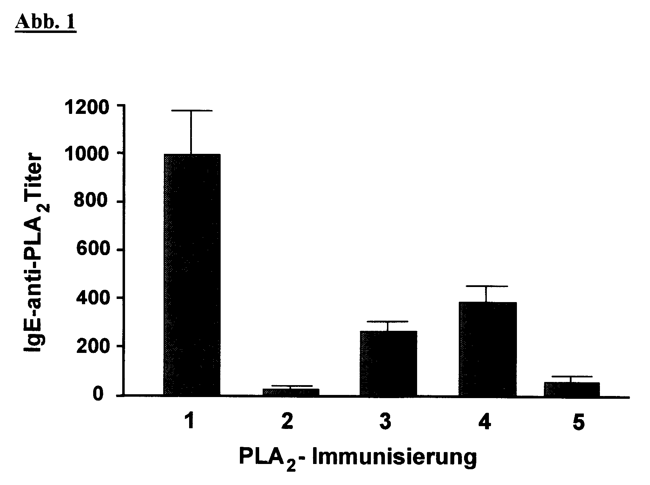

Angegeben sind in Abb. 1 die Titermittelwerte ± Standardabweichung von 4 Tieren pro Versuchsgruppe.

### Beispiel 2:

### Suppression der sekundären Immunantwort durch PLA₂-IgG-Immunkomplexe.

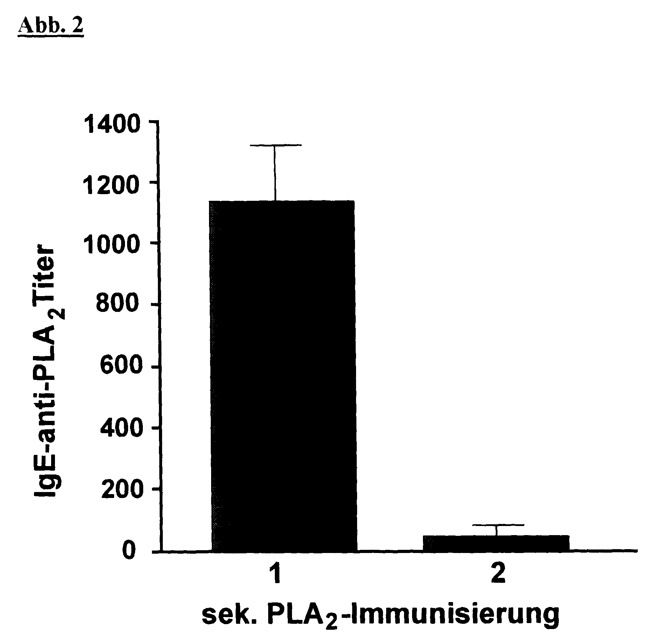

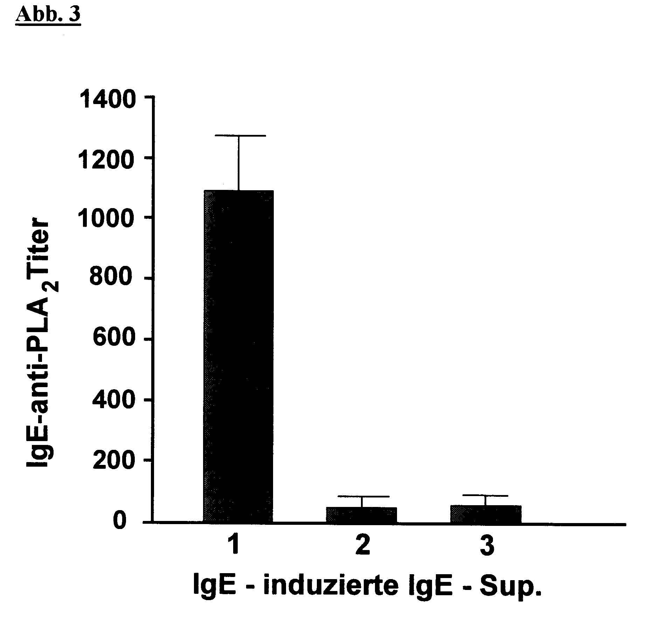

CBA/J-Mäuse wurden primär immunisiert: entweder allein mit 0,1 µg PLA₂ oder mit derselben Menge PLA₂ zusammen mit einem monoklonalen IgG2b-anti-PLA₂ Antikörper (molares Verhältnis 1:10, in beiden Gruppen adsorbiert an 0,2 mg AI(OH)₃). Vier Wochen später wurden beide Gruppen mit der maximalen Dosis von 0,1 µg PLA₂ sekundär immunisiert, und zwei Wochen später wurde im Serum der Tiere der IgE-Titer bestimmt. Angegeben sind in Abb. 2 die Titermittelwerte ± Standardabweichung von 4 Tieren pro Versuchsgruppe.

Es zeigte sich,
1.,) daß der durch die primäre Immunisierung erreichte IgE-Titer der Gruppe 1 nicht weiter erhöht wird, und
2.) daß die in der primären Immunisierung durch die Komplexierung der PLA₂ mit dem monoklonalen IgG2b-anti-PLA₂-Antikörper induzierte Suppression der IgE-Antwort durch die sekundäre Immunisierung mit der PLA₂ nicht aufgehoben wurde.

### Beispiel 3:

### Suppression hoher IgE-Anti-PLA₂-Titer durch Anti-PLA₂- und Anti-Hapten-Antikörper.

CBA/J-Mäuse wurden primär mit der IgE-induzierten Dosis von 0,1 µg PLA₂ [adsorbiert an 0,2 mg Al(OH)₃ - auch bei den anderen beiden Gruppen] immunisiert. Nach 6 Wochen, zum Zeitpunkt der höchsten erreichbaren IgE-Titer, wurden Gruppen von 4 Tieren sekundär immunisiert entweder nur mit 0,1 µg PLA₂ (Gruppe 1) oder mit derselben Dosis PLA₂, welche mit dem monoklonalen IgG2b-anti-PLA₂-Antikörper im 10-fachen molaren Überschuß komplexiert wurde (Gruppe 2), oder mit 0,1 µg PLA₂, an welche das Hapten Phenyloxazolon (PhOx) kovalent gekoppelt war und dann mit einem monoklonalen IgG2b-anti-PhOx-Antikörper komplexiert wurde (Gruppe 3). Nach 2 Wochen wurde der IgE-anti-PLA₂-Titer im Serum der Tiere bestimmt. Angegeben sind die Titermittelwerte ± Standardabweichung von 4 Tieren pro Versuchsgruppe.
Es zeigte sich,
1.) daß der hohe IgE-Spiegel durch PLA₂-anti-PLA₂-IgG2b-Immunkomplexe supprimiert werden konnte, und
2.) daß eine solche Suppression des IgE-Titers auch erreicht werden konnte, wenn die PLA₂ mit dem Hapten PhOx beladen wurde und IgG2b-anti-Hapten-Antikörper zur Immunkomplexbildung eingesetzt wurden.

### Beispiel 4

### Beziehung zwischen Hapten-Kopplungsdichte und notwendiger Konzentration der Anti-Hapten-Antikörper

Die Versuche (Beispiel 1-3) wurden mit den erfolgreichen IgG-Anti-Hapten Antikörpern mit verschiedenen Präparationen der PLA₂ wiederholt, die mit unterschiedlichen Konzentration an Hapten beladen wurden.

### Beispiel 5

### Dauer der Anti-Hapten-IgG-induzierten IgE-Suppression

Hier wurde eine größere Gruppe von Tieren mit dem erfolgreichsten IgE-supprimieren-den Ansatz immunisiert. Im monatlichen Anstand werden dann kleinere Gruppen dieser Tiere PLA₂ als Allergen in einer Dosierung immunisiert, daß normale CBA-Mäuse mit einer starken IgE-Anti-PLA₂ Immunantwort reagieren.

## Patentansprüche

1. Arzneimittel enthaltend neben üblichen Träger und Hilfsstoffen mindestens ein haptenisiertes Allergen und Antikörper gegen dieses Hapten als Kombinationspräparat zur gleichzeitigen, getrennten oder zeitlich abgestuften Anwendung.

2. Arzneimittel enthaltend neben üblichen Träger- und Hilfsstoffen einen Komplex aus haptenisierten Allergen mit Antikörpern gegen dieses Hapten.

3. Arzneimittel enthaltend neben üblichen Träger- und Hilfsstoffen haptenisierten Carrier und haptenisiertes Allergen als Kombinationspräparat zur gleichzeitigen, getrennten oder zeitlich abgestuften Anwendung.

4. Arzneimittel enthaltend neben üblichen Träger- und Hilfsstoffen mindestens ein Allergen und Antikörper gegen dieses Allergen als Kombinationspräparat zur gleichzeitigen, getrennten oder zeitlich abgestuften Anwendung.

5. Arzneimittel enthalten neben üblichen Träger- und Hilfsstoffen einen Komplex aus Allergen mit Antikörpern gegen dieses Allergen.

6. Arzneimittel nach Anspruch 1 bis 3 in denen nur eine haptenisierte Allergenspezies verwendet wird.

7. Arzneimittel nach Anspruch 1, 2, 4, 5 oder 6, dadurch gekennzeichnet, daß die Antikörper IgG's sind.

8. Arzneimittel nach Anspruch 1, 2, 4, 5, 6 oder 7, dadurch gekennzeichnet, daß die Antikörper ein oder mehrere monoklonale Antikörper sind.

9. Arzneimittel nach einem der Ansprüche 1, 2, 4-8, dadurch gekennzeichnet, daß die Antikörper zum Subtyp IgG2a/2b der Maus oder dem korrespondierenden human Subtyp zugeordnet sind.

10. Verwendung von haptenisiertem Allergen und Antikörper gegen dieses Hapten zur Herstellung von Arzneimittel für die Behandlung allergener Erkrankungen.

11. Verwendung von Allergen und Antikörper gegen dieses Allergen zur Herstellung von Arzneimitteln für die Behandlung von allergenen Erkrankungen.

12. Verwendung von Antikörpern gegen Allergene zur Herstellung von Arzneimitteln für die Behandlung von allergischen Erkrankungen.
